# EUROPEAN PATENT APPLICATION

(11) **EP 2 258 721 A1**
(43) Date of publication of application: **08.12.2010**
(21) Application number: 09720469.7
(22) Date of filing: 13.03.2009
(51) Int. Cl.: C07K 14/79, A61K 38/16, A61K 47/48, A61P 1/00, A61P 1/02, A61P 1/04, A61P 1/16, A61P 3/06, A61P 3/10, A61P 9/10, A61P 9/12, A61P 13/00, A61P 13/02, A61P 19/02, A61P 19/04, A61P 25/00, A61P 25/04, A61P 25/08, A61P 25/16, A61P 25/18, A61P 25/20

(54) **POLYETHYLENE GLYCOLATED LACTOFERRIN COMPLEX AND METHOD OF PRODUCING THE SAME**

(30) Priority: 14.03.2008 JP 2008066626
(71) Applicant: NRL Pharma, Inc., Kawasaki-shi, Kanagawa 2130012 (JP)
(72) Inventor: SATO, Atsushi, Fujisawa-shi Kanagawa 251-0032 (JP)
(74) Representative: Giovannini, Francesca
(86) International application number: PCT/JP2009/055557
(87) International publication number: WO 2009/113743

(57) **Abstract**

There are provided a complex of lactoferrin with a hydrophilic, linear and non-peptide polymer which has a prolonged life in vivo and a high clinical usefulness due to its reduced antigenicity and imparted pepsin-resistance, and a method of producing the same. There are also provided a lactoferrin complex which sustains the biological activity of natural lactoferrin at a certain ratio, shows a significantly prolonged life in vivo and is superior in clinical usefulness to natural lactoferrin, a method of producing the same and the like.

One aspect of the invention relates to a method of producing a polyethylene glycolated lactoferrin complex wherein linear polyethylene glycol (PEG) or a modified product thereof is covalently bonded to lactoferrin via an amide bond, the method including causing a reaction in a reaction liquid, which contains lactoferrin and a linear PEG derivative having a para-nitrophenyl group, under conditions of allowing formation of an amide group between the para-nitrophenyl group and lactoferrin.

## Description

### Technical Field

The present invention relates to a biologically active complex of lactoferrin with a non-peptide hydrophilic polymer such as polyethylene glycol (PEG), a method of producing the same, and use thereof.

### Background Art

For the purpose of the regulation of the properties of a biopolymer, etc., conjugation of the biopolymer with a non-peptide hydrophilic polymer such as PEG has conventionally been performed (hereinafter, such conjugation may be referred to as "complexation," or "PEGylation" when PEG or its similar compound is used). More specifically, complexation is carried out generally by attaching an active group to the terminal of a non-peptide hydrophilic polymer and then having the active group react with a functional group present on the molecular surface of a protein or the like.

Particularly, the complexation of a protein or a peptide is important, and the partial coverage of the molecular surface of a protein with a chain of a non-peptide hydrophilic polymer has been studied for shielding an epitope of the protein to reduce the antigenicity and immunogenicity thereof, to reduce the incorporation thereof into the reticuloendothelial system etc., or to prevent the recognition and degradation thereof by proteases. It is also known that the in vivo clearance of such complexed substance is delayed, resulting in prolonged in vivo lifetime. On the other hand, it is frequently observed that an active site of such complexed protein or the like is affected by the presence of the non-peptide hydrophilic polymer to result in reduced biological activity.

For example, interferon when complexed with PEG has in vivo lifetime prolonged about 70-fold but shows reduced biological activity such as antiviral activity to about 1/10. From a comprehensive viewpoint, however, the complexation of interferon with PEG is known to result in significant improvement in its therapeutic effect and is useful for the treatment of hepatitis C.

In the concept of protein complexation, there has been a long history since the successful complexation of asparaginase with PEG which is made for use of the enzyme as a therapeutic agent for leukemia. Until now, the structures of complexing reagents such as PEG (type of the active group, the size and distribution of the molecule, development of branched type, etc.) have been improved and the technologies are advancing.

Complexes of certain proteins with branched PEG are known to have higher protease resistance than the complexes with linear PEG, and to have increased stability against pH and heat depending on the protein (Non-Patent Document 1: Monfardini et al., Bioconjug Chem. 1995 6 (1): 62-9). As to interferon, a complex thereof with branched PEG has a higher anti-proliferative activity than the complex with other types of PEG or the interferon itself (Patent Document 1: Japanese Patent Application Laid-Open (JP-A) No. H10-67800).

However, a fluctuation in the activity of individual proteins upon complexation varies from protein to protein. Further, complexation of a certain protein with a PEG can bring about various influences on many properties of the protein. For example, complexation of interferon with a PEG causes a decrease in its in vitro antiviral activity and an increase in its anti-proliferative activity in human tumor cells. Accordingly, the optimum conditions and the like for obtaining a complex having desired properties should be sufficiently examined for each protein.

It can be easily anticipated that depending on the structure of a chain (linear or branched chain, molecular size, distribution and the like) of a non-peptide hydrophilic polymer, the reaction sites and the number of reacting molecules, the complexation of proteins and the like exerts various influences on biochemical and pharmaceutical properties such as antigenicity, protease resistance, in vivo lifetime and heat stability, and on biological activities involved in pharmaceutical efficacy. Accordingly, when such complexes are to be developed as pharmaceutical preparations, a non-peptide hydrophilic polymer chain should be added at a certain site or sites in order to guarantee certain qualities.

Lactoferrin (hereinafter abbreviated sometimes to "LF") is a glycoprotein with a molecular weight of about 80,000 occurring mainly in mammalian milk and also found in neutrophils, tears, saliva, nasal discharge, bile juice, semen and the like. Lactoferrin binds iron and thus belongs to the transferrin family. Known physiological activities of lactoferrin include an antibacterial activity, an iron metal metabolism regulating activity, a cell growth activating activity, a hematopoietic activity, an anti-inflammatory activity, an antioxidant activity, a phagocytosis promoting activity, an antiviral activity, a bifidobacteria growth promoting activity, an anti-cancer activity, a cancer metastasis inhibiting activity and a translocation inhibiting activity, etc.. Recently, it is revealed that lactoferrin also has a lipid metabolism improving activity, an analgesic/anti-stress activity and an anti-aging activity. As described above, lactoferrin is a multifunctional bioactive protein showing various functions and is expected for use in pharmaceutical preparations and food products for restoration or promotion of health, and lactoferrin-containing food products have already been commercially available.

Lactoferrin, when orally ingested, undergoes hydrolysis by an acid protease, pepsin, present in gastric juice thereby being decomposed into peptides, and thus scarcely arrives as the lactoferrin molecules at the intestinal tract. In the digestive tract, however, lactoferrin receptors are known to be present in the mucosa of small intestine, and it has recently been revealed that lactoferrin is absorbed via the intestinal tract into the body, to express its bioactivity. Therefore, for ensuring the bioactivity of lactoferrin, it is important that lactoferrin is allowed to arrive at the intestinal tract without undergoing hydrolysis by pepsin present in the gastric juice.

With respect to lactoferrin, there is also a report on its PEG complex (Non-Patent Document 2: C. O. Beauchamp et al., Anal. Biochem. 131: 25-33 (1983)). In this document, the PEG used for the PEGlyation reaction was monomethoxy polyethylene glycol (mPEG) with its OH group activated by 1,1'-carbonylimidazole. However, this document merely describes that a complex of the LF with a linear PEG had an in vivo lifetime prolonged 5- to 20-fold, but does not describe the bioactivity of the PEGylated LF, the degree and uniformity of the PEGylation and so on.

In this connection, as a result of intensive studies, the inventors of the present invention have previously succeeded in obtaining a biologically active lactoferrin complex maintaining iron chelating ability of 30% or more of that of natural lactoferrin by having the branched non-peptide hydrophilic polymer complexed to a limited site in the molecular surface of lactoferrin (Patent Document 2). However, no success was made with a linear polymer.

Patent Document 1: Japanese Patent Application Laid-Open (JP-A) No. H10-67800
Patent Document 2: JP-A No. 2007-70277
Non-Patent Document 1: Monfardini et al., Bioconjug Chem. 1995 6 (1): 62-9
Non-Patent Document 2: C. O. Beauchamp et al., Anal. Biochem. 131: 25-33 (1983)

### Disclosure of the Invention

### Problems to be Solved by the Invention

An object of the present invention is to provide a clinically highly useful complex of lactoferrin with a linear and non-peptide hydrophilic polymer which has reduced antigenicity and a prolonged in vivo lifetime attributable to imparted pepsin resistance, as well as a method of producing the same. Another object of the invention is to provide a lactoferrin complex with a certain amount of the bioactivity of natural lactoferrin, a significantly prolonged in vivo lifetime and better clinical usefulness than that of natural lactoferrin, as well as a method of producing the same.

In particular, another object of the invention is to provide a method which is appropriate for efficient preparation of a massive amount of the lactoferrin complex having the characteristics described above within a short period of time.

### Means for Solving the Problems

The present inventors examined reaction conditions and the like for complexing lactoferrin most uniformly with a non-peptide hydrophilic polymer such as a linear PEG while maintaining the biological activity of lactoferrin, thereby enabling such a polymer having a specific structure to be bound to limited sites of the molecular surface of lactoferrin. The inventors also found the conditions and methods that are appropriate for mass preparation or industrial production of the complex. The inventors obtained the results that the thus produced lactoferrin complex had resistance to proteases such as pepsin and the like, while maintaining an iron-chelating ability that is the most important bioactivity and the ability of controlling (inhibiting) the production of inflammatory cytokines. As a result, the invention was thereby completed.

That is, the present invention provides the following items:
[1] A method of producing a polyethylene glycolated lactoferrin complex, wherein a linear polyethylene glycol (PEG) or a modified product thereof is covalently bonded to lactoferrin via an amide bond, the method comprising:
   causing a reaction to occur in a reaction liquid, comprising the lactoferrin and a linear PEG derivative having a para-nitrophenyl group, under conditions that allow formation of an amide group between the para-nitrophenyl group and the lactoferrin.
[2] The method according to the above item [1], wherein the reaction step is carried out under the condition of pH 4 to 5.5.
[3] The method according to the above item [1] or [2], wherein the reaction step is carried out under the conditions that a molar ratio of the lactoferrin to the linear PEG derivative is 1:1 to 1:5, a final concentration of the lactoferrin is 5 mg/ml to 10 mg/ml, and a reaction time is 24 to 48 hours.
[4] A PEGylated lactoferrin complex produced by the method according to any of the above items [1] to [3], wherein a linear polyethylene glycol (PEG) or a modified product thereof is covalently bonded to the lactoferrin via an amide bond.
[5] A pharmaceutical composition including:
   the PEGlyated lactoferrin complex according to the above item [4]; and
   a therapeutically inert base and/or an additive.
[6] The pharmaceutical composition according to the above item [5], which is to be orally administered.

### Effects of the Invention

According to the method of producing a PEGylated lactoferrin complex of the invention, by using linear PEG derivatives, a target lactoferrin complex, in which the PEG is uniformly added, can be efficiently and readily produced within a short period of time. According to the production method of the invention, it is assumed that the reaction occurs uniformly only at limited sites having high reactivity. As a result, a population of very uniform complexes is obtained.

The complex of the invention maintains the ability of lactoferrin to bind to iron, and thus continues to have at least the lactoferrin's important bioactivity based on the iron binding ability. Because the complex of the invention has been endowed with resistance to proteases such as pepsin and the like by the binding to the linear PEG derivatives, the complex has a long in vivo lifetime and can exhibit the bioactivity for a long time in the body. Further, lactoferrin is made less susceptible to digestion and degradation by pepsin in the stomach as a result of complexation, and thus, it can reach the intestine sufficiently without needing any further pharmaceutical treatment for dissolution in the intestine. The complex of the invention also fully maintains the activity of controlling the production of inflammatory cytokines.

The complex of the invention is a complex wherein a constant number of PEGs are bound to specific sites of lactoferrin. Thus, the complex is uniform in qualities and advantageous in terms of production and quality controls. Based on these advantages, the complex of the invention is suitable for use as a pharmaceutical ingredient, especially as an orally-administered pharmaceutical ingredient. That is, lactoferrin can be made in the form which is more useful as a pharmaceutical ingredient by the complex of the invention and the method of producing the complex. Lactoferrin is extremely safe and has various bioactivities, and is thus further advantageously applicable by the invention as a therapeutic or prophylactic agent for diseases or symptoms for which there is no effective therapeutic agent. For example, the complex of the invention can be applied to a broader spectrum of applications for lifestyle-related diseases (arteriosclerosis, hypercholesterolemia, hyperlipidemia, hypertension, diabetes mellitus, steatosis etc.), cancers (cancer prevention, secondary prevention of cancer, metastasis suppression, enhancement of anticancer agent activity, etc.), autoimmune diseases (dry eye and dry mouth resulting from Sjogren's syndrome, rheumatoid arthritis, malignant rheumatoid arthritis, collagen disease, multiple sclerosis, systemic lupus erythematosus etc.), neuropsychiatric disorders (dementia, Alzheimer's disease, Parkinson's disease, epilepsy, depression, social withdrawal, schizophrenia, various stress-related illnesses etc.), pain relief (enhancing activity of opioid such as morphine, cancer-related pain, neuropathic pain, postherpetic pain, fibromyalgia, postoperative pain, glossodynia, cramps, toothache, arthralgia etc.), hepatitis (various types of viral hepatitis, non-alcoholic hepatitis, hepatic cirrhosis etc.), inflammatory bowel diseases (colon ulcer, Crohn's disease etc.), the irritable bowel syndrome, prostatic hyperplasia, pollakiuria, insomnia and constipation, etc.

Furthermore, lactoferrin contained in the complex of the invention has an antibacterial/antiviral activity and an immunity activating activity, and thus the complex of the invention and a pharmaceutical composition containing the complex can also be applied to various infections and inflammations based thereon, for example, gastric mucosal infection with Helicobacter pylori, periodontal diseases, alveolar pyorrhea, halitosis, oral candidiasis, stomatitis, angular stomatitis, rhinitis, esophagitis, cholecystitis, urinary tract infection, vaginal infection, tinea pedis, acne, herpes group virus infection, senile pneumonia and postoperative infection, and it also has an activity of enhancing the activity of antibiotics.

Meanwhile, lactoferrin also has an activity of introducing immunological tolerance, so the complex of the invention and a pharmaceutical composition containing the complex can also be used against allergic diseases such as pollinosis, atopic dermatitis, seborrhea and urticaria, etc. It should be noted that lactoferrin has a potent antioxidant stress activity based on the iron chelating activity, and the complex of the invention and a pharmaceutical composition containing the complex can also be applied to Wilson's disease, fulminant hepatic failure, anti-aging and rejuvenation of the skin and eye, age-related macular degeneration, diabetic retinopathy, and keratinization suppression and rejuvenation of mucosal epithelial cells, etc.

### Brief Description of the Drawings

Fig. 1 is a schematic diagram showing a reaction between a linear PEG derivative having a para-nitrophenyl group as a functional group and bLF. Panel A indicates the linear PEG derivative and LF before the reaction and panel B indicates a complex and byproducts after the reaction.
Fig. 2 is a gel photograph showing analysis on complexation between the linear PEG derivative having an NHS ester as a functional group and the bLF. The band indicated by the arrow represents unmodified bovine lactoferrin. Panels A, B and C show results obtained by using a PEGylating reagent, "Methoxy polyethylene glycol succinimidyl succinate", MW=5 kDa, manufactured by Sigma Chemical Corporation; a PEGylating reagent, "SUNBRIGHT MEGC-30TS", MW=30 kDa, manufactured by NOF Corporation; and a PEGylating reagent, "SUNBRIGHT ME-200TR", MW=20 kDa, manufactured by NOF Corporation, respectively.
Fig. 3 is a gel photograph showing analysis on complexation between a linear PEG derivative ("SUNBRIGHT MENP-50H", (5 kDa)) having a para-nitrophenyl group and a bLF (5K-PEG-Lf) under different pH conditions.
Fig. 4 is a gel photograph showing analysis on complexation between a linear PEG derivative ("SUNBRIGHT MENP-30T", (30 kDa)) having a para-nitrophenyl group and a bLF (30K-PEG-Lf) under different pH conditions.
Fig. 5 is a gel photograph showing analysis on formation of 5K-PEG-Lf under various reaction time conditions at 25°C.
Fig. 6 is a gel photograph showing analysis on formation of 30K-PEG-Lf under various reaction time conditions at 25°C.
Fig. 7 is a gel photograph showing analysis on 5K-PEG-Lf, which has been purified by a cation exchanger and analyzed by 7.5% SDS-PAGE.
Fig. 8 is a gel photograph showing analysis on 30K-PEG-Lf, which has been purified by a cation exchanger and analyzed by 7.5% SDS-PAGE.
Fig. 9 is a gel photograph showing unmodified lactoferrin (bLf) and purified 30k-PEG-Lf, which have been digested with pepsin and analyzed by 10% SDS-PAGE.
Fig. 10 is a diagram in which degradation of 30k-PEG-Lf with time by pepsin is compared with the degradation of unmodified bLF. ◆ corresponds to bLF and ■ corresponds to 30k-PEG-Lf.

### Best Mode for Carrying out the Invention

The complex of the invention is a biologically active complex between a linear PEG and lactoferrin. Derivatives of the linear PEG which are to be bound to lactoferrin to form the complex of the invention may be a polymer having, at one terminal thereof, a para-nitrophenyl group capable of allowing a reaction with a functional group of lactoferrin to form a covalent bond therebetween, and which polymer being compatible to the living body or physiologically inert.

The complex of the invention is expressed by the following formula.

Formula (I): [PEG]-CONH-[LF]

(wherein "[PEG]" represents polyethylene glycol (HO-(CH₂-CH₂-O)ₙ-) or its modified product, "CONH" represents an amide bond, and "[LF]" represents lactoferrin).

In Formula (I), the [PEG] moiety indicates PEG or its modified product (for example, methoxylated PEG), and may have a short branch or a pendant group. Linear PEG or methoxylated PEG is preferable, in particular.

The "lactoferrin" (LF) used in the complex of the invention may be a naturally occurring or natural-type lactoferrin molecule itself or lactoferrin obtained by genetic recombination (including modified lactoferrin by partial amino acid replacement) or a functional equivalent of lactoferrin, such as an active fragment of lactoferrin, and is not limited by the presence or the absence of iron ions, the content of iron ions, the biological species from which lactoferrin is derived, and the like.

In naturally occurring lactoferrin, there are about 44 (human LF) to 54 (bovine LF) lysine residues, but the reactivity of each residue varies depending on the local environment of the position where the residue is present. According to the method of the invention, the α-amino group at the N-terminal and the ε-amino group of a lysine residue in lactoferrin are the target site for modification by PEG However, in the complex, non-peptide hydrophilic polymers are covalently bounded, with good reproducibility, to 1 to 10 functional groups, preferably 1 to 5 functional groups. In particular, by carrying out the process under the optimum condition, highly uniform and selective PEGylation, like 1 functional group per LF molecule, is achieved.

The term "biologically active" described in connection with the complex of the invention means that the physiological and pharmacological activity of lactoferrin is maintained. Particularly, the complex of the invention has an iron chelating (binding) ability and/or an ability of controlling inflammatory cytokines that are almost equivalent to that of naturally occurring lactoferrin.

Specifically, when the iron binding ability of naturally occurring lactoferrin, as determined by the method described in the Examples below, is assumed to be 100%, the complex of the invention maintains at least 50% or more (for example, about 50% to about 150%, or about 50% to about 120%) iron binding ability. In a preferable embodiment, the complex of the invention has about 60% to about 100% or more (for example, about 60% to about 150%, or about 60% to about 120%) of the iron binding ability of naturally occurring lactoferrin. When the iron binding ability is measured by the method described in the Examples or a method equivalent thereto, there may be an error of about ±20%.

Furthermore, when the ability of controlling the production of inflammatory cytokines of naturally occurring lactoferrin, as determined by the method described in the Examples below, is assumed to be 100%, the complex of the invention maintains at least 50% (for example, about 50% to about 150%, or about 50% to about 120%) of the ability of controlling the production of inflammatory cytokines. In a preferable embodiment, the complex of the invention has about 70% to about 100% or more (for example, about 70% to about 150%, or about 70% to about 120%) of the ability of controlling the production of inflammatory cytokines of naturally occurring lactoferrin. When the ability of controlling the production of cytokines is measured by the method described in the Examples or methods equivalent thereto, there may be an error of about ±20%.

The complex of the invention has protease resistance. That is, the complex of the invention, as compared with naturally occurring lactoferrin, is significantly resistant to digestion with at least pepsin and/or trypsin or chymotrypsin. Preferably, the complex of the invention has such pepsin resistance that after digestion with pepsin for 20 minutes under the conditions described in the Examples, the lactoferrin remains undigested at a higher degree by about 1.5- to about 2-fold or more (for example, about 2- to about 5-fold) than naturally occurring lactoferrin does.

The complex of the invention can be produced by establishing a covalent bond based on the reaction between the para-nitrophenyl group of a linear PEG derivative and the functional group of lactoferrin. As the linear PEG derivative, for example, those indicated by the following formula (II) can be used.

Formula (II): [PEG]-[PNP]

(wherein "[PEG]" represents polyethylene glycol or its modified product, and "[PNP]" represents a para-nitrophenyl group).

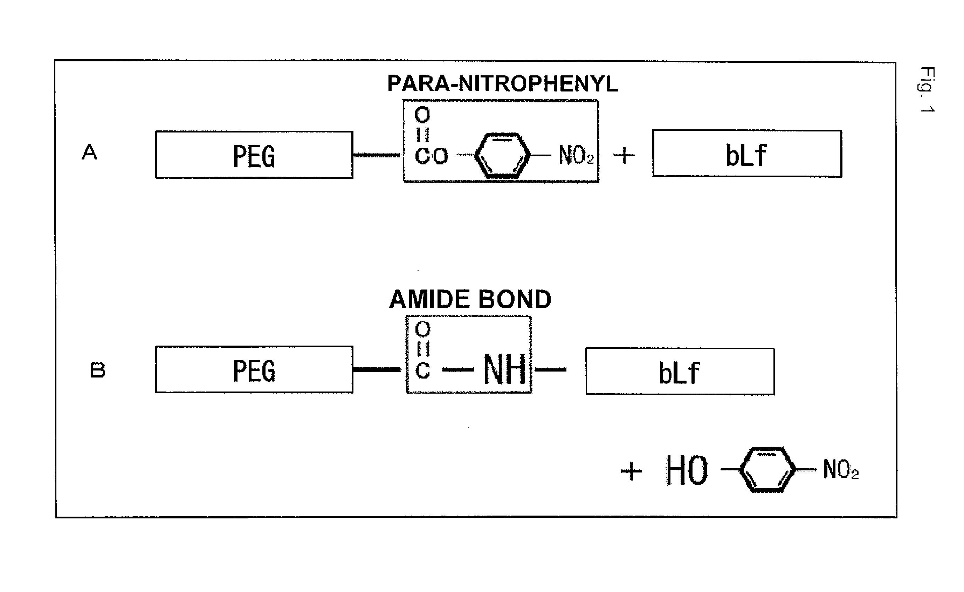

Schematic diagram showing the reaction in which a linear PEGylating reagent having a para-nitrophenyl group as a functional group is reacted with bovine LF (bLf) to form the PEGylated lactoferrin complex of the invention is given in Fig. 1 (Before the reaction: Panel A; After the reaction: Panel B).

Such a linear PEG derivative may be synthesized by methods known in the art, or may be selected from a wide variety of commercially available products. The molecular weight (number-average molecular weight) of the linear PEG derivative used in the reaction is generally about 500 to about 200,000, preferably about 2,000 to about 100,000, and particularly preferably about 10,000 to about 60,000 (Da).

Preferably, for the PEGylation reaction, lactoferrin and the linear PEG derivative are added in a molar ratio of 1:0.1 to 1:100 to a reaction liquid. The mixing molar ratio of lactoferrin to the linear PEG derivatives is more preferably in the range of 1:0.1 to 1:60, and most preferably in the range of 1:1 to 1:5.

In addition, the reaction step is generally carried out under the conditions in which pH is 4 to 5.5, temperature is 0°C to 40°C, and time is 1 minute to 72 hours. pH of the reaction liquid is more preferably pH 5±0.5, and most preferably near pH 5 (i.e., pH 4.75 to 5.25).

The reaction time and the reaction temperature may be varied in a closely related manner. In general, it is preferable that the reaction time is short when the reaction temperature is high, and the reaction time is long when the reaction temperature is low. For example, under the condition such that the molar ratio of lactoferrin to the linear PEG derivative is 1:1 to 1:5, when the reaction is carried for about 1 to 48 hours at 25°C, particularly favorable result (e.g., uniform complexation, etc.) can be obtained.

The complex of the invention contained in a sample, which is produced as described above, can be easily purified by first performing adsorption using a cation exchange carrier (resin) for concentration and then applying the resulting concentrate onto a molecular sieve chromatography carrier (resin). Specifically, a sample containing the complex is first applied onto a cation exchange column so that the complex is adsorbed onto the column, and then the complex is eluted with a buffer having a high salt concentration to collect eluates containing the concentrated complex. Then, the eluates may be applied onto a molecular sieve chromatography column and then desalted, and the buffer in the eluates can be replaced with a desired buffer. If necessary, the eluates may further be concentrated suitably by known methods such as dialysis and ultrafiltration, etc.

The purification of the complex of the invention may be carried out by, instead of the above two-step column chromatography, a way of one-step purification using only a cation exchange column. For example, only by applying a sample containing the complex of the invention to a cation exchange column so that the complex is adsorbed onto the column and then by eluting it with a buffer having a high salt concentration, sufficient separation of the complex from unreacted lactoferrin may be achieved.

For the elution from the cation exchanger, it was found that the PEGylated lactoferrin was selectively eluted at the salt concentration of 0.3 M to 0.45 M. Thus, while elution can be performed based on the linear concentration gradient or the step-wise elution, as for the eluent, a concentration gradient or a solution containing the salt concentration of 0.3 M to 0.45 M is used. An elution method based on the step-wise salt concentrations is more convenient and very useful for large-scale purification compared to an elution method based on the linear salt concentration gradient. Thus, for industrial production or mass preparation, an elution method based on step-wise salt concentrations is more advantageous.

Carriers for the cation exchanger which can be used for the purification method of the invention include silica gel. Due to its high physical strength, silica gel is appropriate for industrial production or mass preparation.

Lactoferrin has a wide variety of bioactivities including an antibacterial activity, an iron metal metabolism regulating activity, a cell growth activating activity, a hematopoietic activity, an anti-inflammatory activity, an antioxidant activity, a phagocytosis-promoting activity, an antiviral activity, a bifidobacteria growth promoting activity, an anticancer activity, a cancer metastasis inhibiting activity, a translocation inhibiting activity, a lipid metabolism improving activity, an analgesic activity and an anti-stress activity, and enables, by these activities, the treatment (including amelioration) and prevention of many diseases, or symptoms thereof, including lifestyle-related diseases (for example, hypercholesterolemia and hyperlipidemia), pain control (for example, cancerous pain and neuropathic pain), collagen diseases (for example, dry eye and dry mouth resulting from Sjogren's syndrome, and rheumatic arthritis), periodontal diseases and hepatitis C.

As the complex of the invention sufficiently maintains the bioactivities of lactoferrin such as iron binding activity and an activity of controlling the production of inflammatory cytokines while exhibiting no cell toxicity, it may be formed into a pharmaceutical composition with a therapeutically inert base and/or an additive. Furthermore, as lactoferrin is incorporated via the intestinal mucosa and the complex of the invention is resistant to pepsin, it is advantageous that the complex is prepared in the form of a pharmaceutical composition for oral administeration.

For the sake of convenience, a pharmaceutical preparation or pharmaceutical composition as used herein in relation to the invention encompasses preparations and compositions whose administration subject is an animal (that is, veterinary medicines etc.) in addition to those for humans. Various components which can be contained in such pharmaceutical compositions and formulation types are known to those skilled in the art.

The effective dose of the pharmaceutical composition containing the complex of the invention varies depending on the type or severeness of diseases or symptoms to be treated or prevented, the condition of a subject to whom the composition is administered and the formulation type, etc., and it can be selected suitably in consideration of the known effective dose of lactoferrin as a guide. Generally, the dose may be significantly reduced (for example, 1/2 to 1/20 in terms of the amount of lactoferrin) relative to the known effective dose of lactoferrin, and when used in the same dose as the known dose, the frequency of administration can be reduced.

### Examples

### 1. PEGylation of bovine lactoferrin (bLF)

### 1-1. Reagents

Bovine lactoferrin (bLF) was obtained from MG Nutritionals (Australia) and used. The target for PEGylation was lysine ε-amino groups (54 groups are present per bLF molecule) and the N-terminal α-amino group of bLF.

As for the PEG derivatives used, three types of linear PEG derivatives having an N-hydroxysuccinimide (NHS) activated ester as a functional group (herein below, referred to as "PEG-NHS derivatives": "Methoxy polyethylene glycol succinimidyl succinate", MW=5 kDa, manufactured by Sigma Chemical corporation; "SUNBRIGHT ME-200TR", MW=20 kDa, manufactured by NOF Corporation; and "SLUNBRIGHT MEGC-30TS", MW=30 kDa, manufactured by NOF Corporation), and two types of linear PEG derivatives having a para-nitrophenyl group as a functional group (herein below, referred to as "PEG-PNP derivatives": "SUNBRIGHT MENP-50H", MW=5 kDa, manufactured by NOF Corporation and "(SUNBRIGHT MENP-30T", MW=30 kDa, manufactured by NOF Corporation, were used.

### 1-2. Reaction between PEG-NHS derivatives and bLF

0.5 mg of bovine lactoferrin (bLf) (6.25 µM) dissolved in 1 ml of PBS (pH 7.4) was admixed with the PEG-NHS derivatives in the molar ratio given below. Coupling reaction was carried out at 25°C for 1 hour. The final concentration of lactoferrin was 0.5 mg/ml. Molar ratio of PEG-NHS derivatives to the lysine residues was varied in the range of 1:5 (1.69 mM) to 1:1 (337.5 µM) and the molar ratio of PEG-NHS derivatives to the protein was varied in the range of 1:25 (156.25 µM) to 1:1 (6.25 µM). Coupling reaction was evaluated by Coomassie Brilliant Blue (CBB) staining following SDS-PAGE.

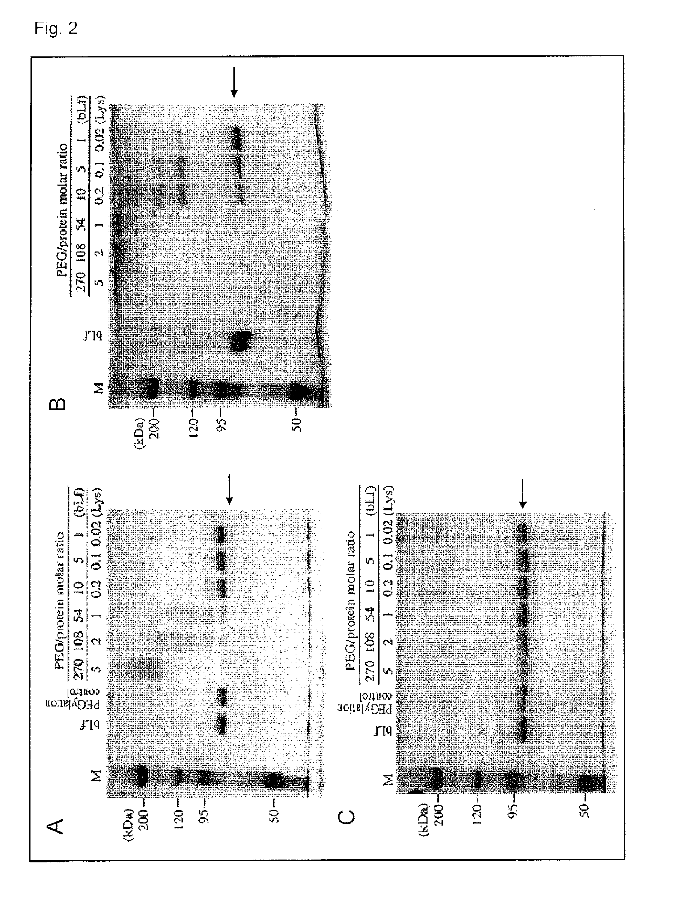

The results are shown in Fig. 2. Panels A, B, and C indicate the results obtained by using the PEGylating reagent "Methoxy polyethylene glycol succinimidyl succinate", MW=5 kDa, manufactured by Sigma Chemical Corporation; the PEGylating reagent "SUNBRIGHT MEGC-30TS", MW=30 kDa, manufactured by NOF Corporation; and the PEGylating reagent "SUNBRIGHT ME-200TR", MW=20 kDa, manufactured by NOF Corporation, respectively. When the coupling reaction was carried out by using "Methoxy polyethylene glycol succinimidyl succinate" manufactured by Sigma Chemical Corporation (Panel A) or "SUNBRIGHT MEGC-30TS" manufactured by NOF Corporation (Panel B), lactoferrin modified with several to many PEGs was obtained. On the other hand, when "SUNBRIGHT ME-200TR" manufactured by NOF Corporation was used, the reactivity was poor and no PEGylated lactoferrin was identified after Coomassie staining (Panel C).

Thus, it was found that the PEG-NHS derivatives had low reaction selectivity and selectively PEGylated lactoferrin could not be prepared by using the linear PEGylating reagent having an NHS activated ester as a functional group.

### 1-3. Reaction between PEG-PNP derivatives and bLF

Reaction between PEG-PNP derivatives and bLF was performed under various conditions. The coupling reaction was carried out under the conditions that the final concentration of bLF was 0.5 mg/ml, the mixing ratio of bLF to PEG derivatives was 1:5, the reaction temperature was 25°C, and the reaction time was 1 hour or 24 hours, with the pH of the solution for PEGylation reaction varied from 4 to 9. The buffers used were a sodium acetate butter for pH 4 to 5, a phosphate buffer for pH 6 to 8, and a borate buffer for pH 9. The final concentration of each buffer was 50 mM. After the reaction, the reaction product was analyzed by 7.5% SDS-PAGE and CBB staining.

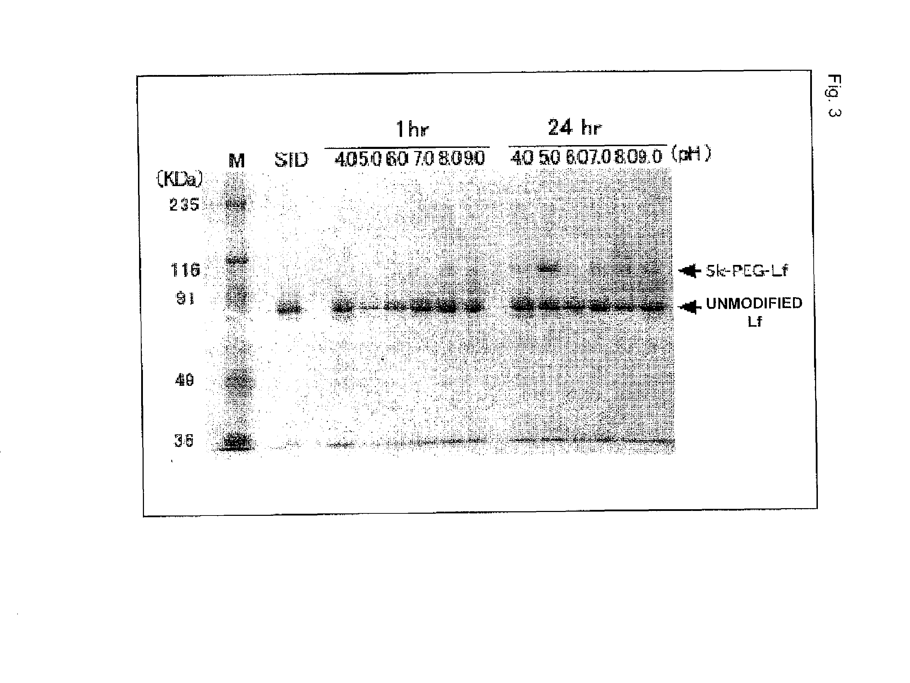

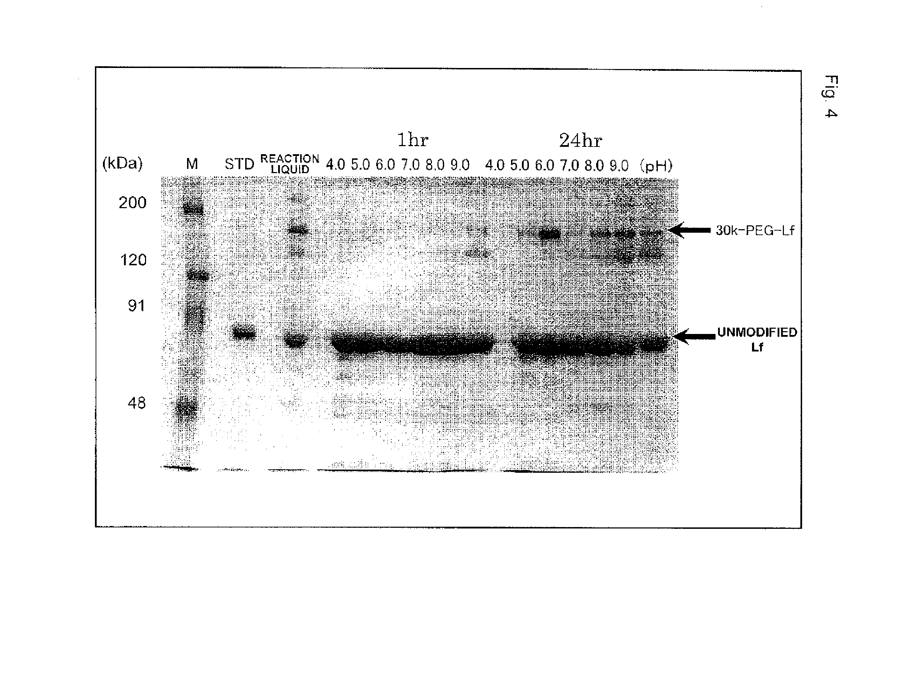

The results obtained using, as the PEGylating reagent, "SUNBRIGHT MENP-50H" (5 kDa) and "SUNBRIGHT MENP-30T" (30 kDa) are shown in Figs. 3 and 4, respectively. It is found that almost no reaction occurred in any pH condition at 1 hour of reaction time. On the other hand, at 24 hours of reaction time, particularly selective and highly productive PEGylation was confirmed in the reaction at pH 5 using "SUNBRIGHT MENP-50H" (5 kDa) or "SUNBRIGHT MENP-30T" (30 kDa).

Herein below, the lactoferrin that is PEGylated with "SUNBRIGHT MENP-50H" (5 kDa) is called "5k-PEG-Lf," and the lactoferrin that is PEGylated with "SUNBRIGHT MENP-30T" (30 kDa) is called "30k-PEG-Lf."

### 2. Determination of mixing molar ratio between bLF and PEGylating reagent and reaction time

It is demonstrated from section 1-3 above that the optimum pH for PEGylation reaction is pH 5. Next, the molar ratio of bLF to PEG-PNP derivatives at the time of reaction of 1:1, 1:3 or 1:5, the final bLF concentration of 0.5 mg/ml, 5 mg/ml or 10 mg/ml, and the reaction time of 24 hours, 48 hours or 72 hours were examined to optimize the conditions for PEGylation. The buffers used in the reaction solutions were 50 mM sodium acetate butter (pH 5.0) for the experiments using "SUNBRIGHT MENP-50H"(5 kDa) and 10 mM sodium acetate butter (pH 5.0) for the experiments using "SUNBRIGHT MENP-30T" (30 kDa).

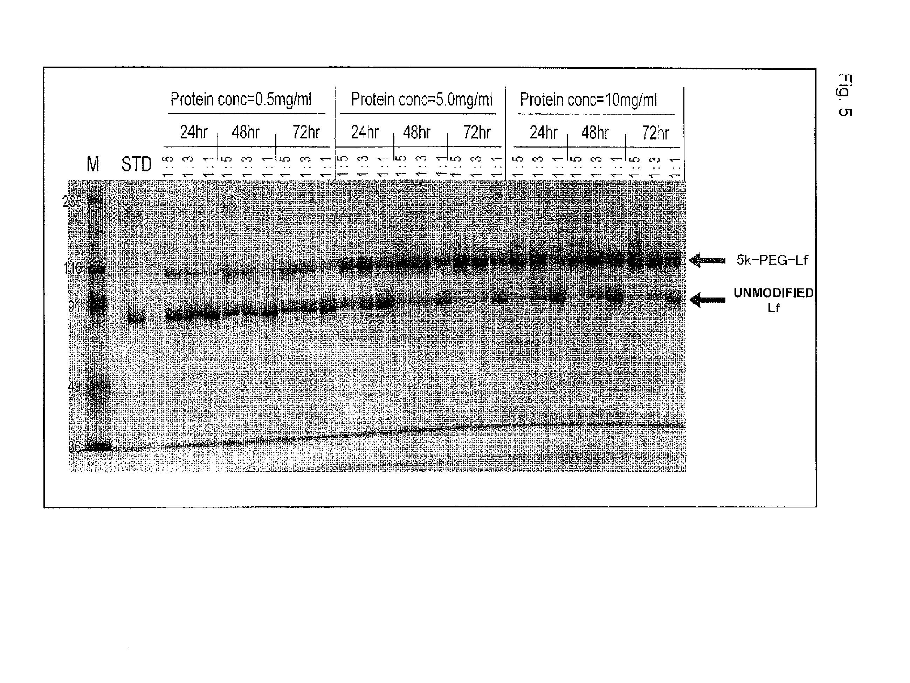

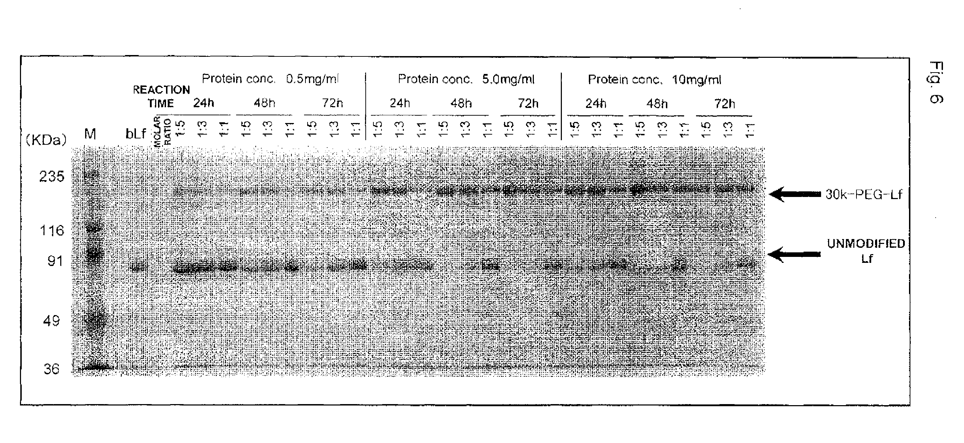

The results obtained with "SUNBRIGHT MENP-50H" (5 kDa) and "SUNBRIGHT MENP-30T" (30 kDa) are shown in Fig. 5 and Fig. 6, respectively. According to the determination of the final bLF concentration, a greater amount of the PEGylated product was obtained at 5 mg/ml and 10 mg/ml compared to 0.5 mg/ml. Furthermore, in every reagent used, it was confirmed that better progress of the reaction was obtained after 48 hours and 72 hours compared to 24 hours. However, when the reaction products of 48 hours and 72 hours are compared to each other, the concentration of the reaction product band was almost identical. Thus, it is believed that the PEGylation reaction was completed within 48 hours.

From the results above, in addition to the pH condition, it is concluded that the conditions for the optimum PEGylation reaction include the molar ratio of bLF to PEG derivatives of 1:1 to 1:5, the final concentration of bLF of 5 mg/ml to 10 mg/ml, and the reaction time of 24 hours to 48 hours for both "SUNBRIGHT MENP-50H" (5 kDa) and "SUNBRIGHT MENP-30T" (30 kDa).

### 3. Purification of 5k-PEG-Lf

By using a cation exchange column carrier (trade name: "MacroCap SP", manufactured by GE Healthcare), the PEGylated lactoferrin (5k-PEG-Lf) was purified from the reaction liquid containing PEGylated bLF.

150 mg of bLF and 46.9 mg of the PEGylating reagent (SUNBRIGHT MENP-50H) were reacted in 30 ml of 50 mM sodium acetate buffer (pH 5.0) at 25°C for 48 hours (the protein concentration was 5 mg/ml and the molar ratio of the protein to the PEGylating reagent was 1:5). The reaction liquid was adsorbed to 3 ml of MacroCap SP for purification. As a buffer, 10 mM sodium phosphate buffer (pH 7.6) was used and the elution of the material adsorbed to the column carrier was carried out by an elution method based on the serial salt concentration gradient by using the buffer containing 0.3 M, 0.4 M, 0.45 M or 1.0 M NaCl. The collected fractions were subjected to 7.5% SDS-PAGE, and then identified by CBB staining.

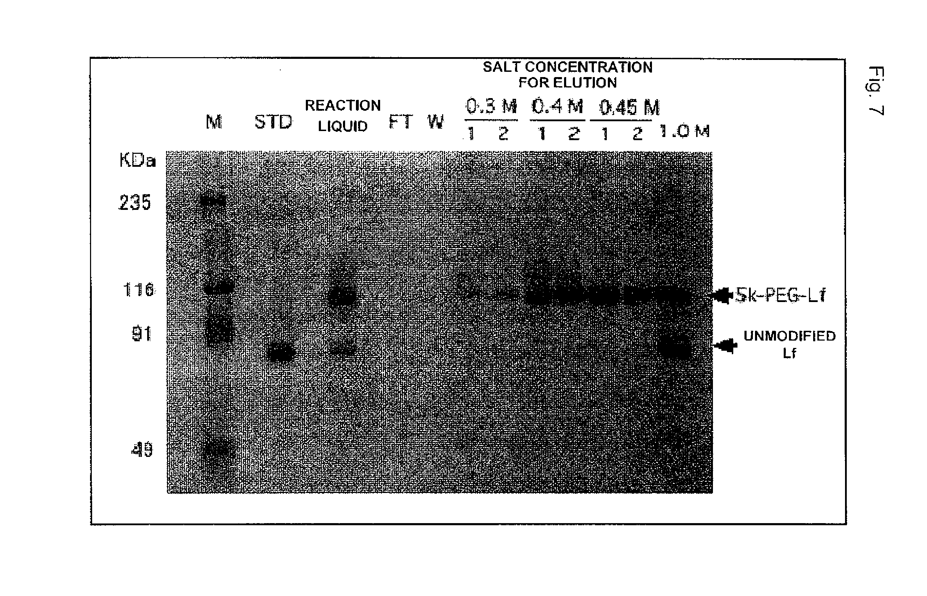

Results are shown in Fig. 7. It was confirmed that only the PEGylated lactoferrin was purified in the eluate fractions of 0.3 M, 0.4 M or 0.45 M NaCl.

### 4. Purification of 30k-PEG-Lf

By using a cation exchange column carrier (trade name: "MacroCap SP", manufactured by GE Healthcare), the PEGylated lactoferrin (30k-PEG-Lf) was purified from the reaction liquid containing PEGylated bLF.

200 mg of bLF and 375.2 mg of the PEGylating reagent (SUNBRIGHT MENP-30T) were reacted in 40 ml of 50 mM sodium acetate buffer (pH 5.0) at 25°C for 48 hours (the protein concentration was 5 mg/ml and the molar ratio of the protein to the PEGylating reagent was 1:5). The reaction liquid was absorbed to 3 ml of "MacroCap SP" for purification. As a buffer, 10 mM sodium phosphate buffer (pH 7.6) was used and the elution of the material adsorbed to the column carrier was carried out by an elution method based on the serial salt concentration gradient by using the buffer containing 0.4 M, 0.45 M or 1.0 M NaCl. The collected fractions were subjected to 7.5% SDS-PAGE, and then identified by CBB staining.

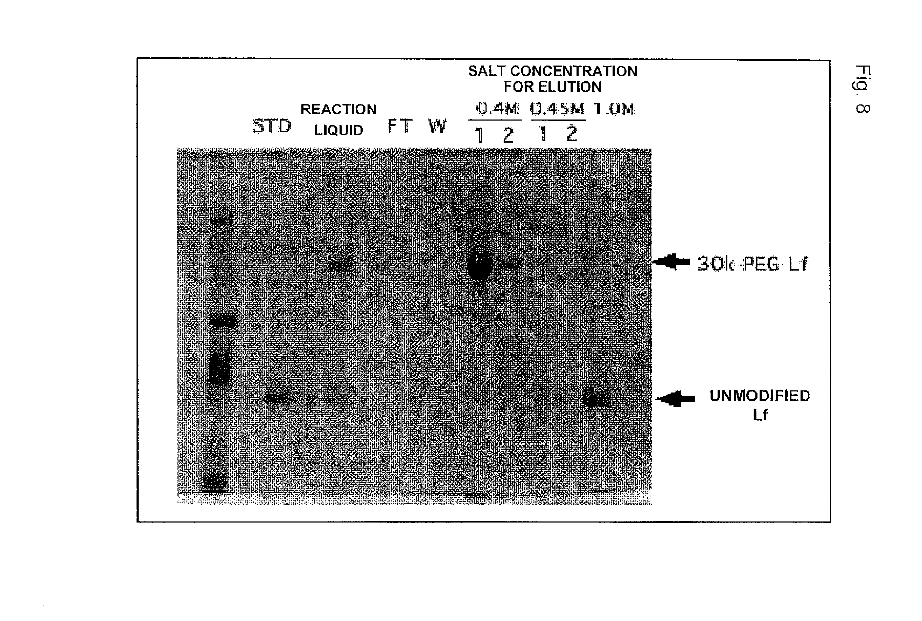

Results are shown in Fig. 8. It was confirmed that only the PEGylated lactoferrin was purified in the eluate fractions of 0.4 M NaCl.

### 5. Anti-inflammatory activity of 5k-PEG-Lf and 30k-PEG-Lf (activity of inhibiting IL-6 production)

An anti-inflammatory activity is one of the physiological activities of bLF. It has been reported that, in human monocyte (THP-1) culture system, bLF inhibits the production of IL-6 caused by endotoxin shock of lipopolysaccharides (LPS) (Mattsby-Baltzer I et al., Pediatr Res, 40, 257-262 (1996) and Haversen L et al., Cell Immunol, 220, 83-95 (2002)). In this connection, 5K-PEG-Lf and 30k-PEG-Lf, which had been prepared and purified in the above sections 3 and 4, were tested for the anti-inflammatory activity.

THP-1 cells which have been successively subcultured in RPMI1640 medium (supplemented with 10% FBS and 2 mM GlutaMAX (tradename, manufactured by Invitrogen Corporation); herein below referred to as "the medium") were subcultured at the cell concentration of 5 × 10⁴ cells/ml. After culturing for 24 hours, human IFN-γ (40 ng/ml) and calcitriol (20 ng/ml) were added and the cells were further cultured at 37°C, 5% CO₂ for 48 hours. Cultured cells were collected by centrifugation and washed with the medium. Then, the cells were suspended in the fresh medium to obtain the cell suspension having a concentration of 1 × 10⁶ cells/ml. The cell suspension was dispensed in a 24-well plate (400 µl/well). After keeping the plate for 1 hour at 37°C, 5% CO₂, one of the followings, i.e., LPS only (100 ng/ml), LPS (100 ng/ml)+bLF (100 µg/ml), LPS (100 ng/ml)+5K-PEG-Lf (100 µg/ml), and LPS (100 ng/ml)+30k-PEG-Lf (100 µg/ml), was added to each well and the culture supernatant was collected after 24 hours.

The amount of IL-6 contained in the culture supernatant was measured according to the instructions enclosed in the kit for measuring human IL-6 ELISA measurement (manufactured by Kamakura Techno Science Inc.). The activity of inhibiting IL-6 production was calculated by comparing the amount of IL-6 produced when only LPS was added to the amount of IL-6 produced when both LPS and bLF or the PEGylated LF were added, and determining the amount of IL-6 that was not produced due to the inhibition. Relative activity of 5K-PEG-Lf and 30k-PEG-Lf was calculated using the inhibiting activity of bLF as being 100%.

As s result, 5K-PEG-Lf showed residual activity of 76.8±6.0% (mean±SE, n=8), while 30k-PEG-Lf showed residual activity of 115.3±3.2% (mean±SE, n=6).

### 6. Determination of iron-binding ability of 5k-PEG-Lf and 30k-PEG-Lf

Lactoferrin is a nonheme, iron-binding glycoprotein having a molecular weight of 80,000, consisting of two domains called N-lobe and C-lobe. In the presence of carbonate ion (CO₃²⁻), it has an ability to reversibly chelate two iron ions (Fe³⁺) per protein molecule (Anderson, et al., Nature, 344, 784-78 (1990)). The iron binding ability of lactoferrin was measured in the following manner.

Apo-form lactoferrin was prepared by releasing iron ions (Fe³⁺) from holo-form lactoferrin. Then, iron ions (Fe³⁺) were added to the lactoferrin in the presence of carbonate ions (CO₃²⁻) to prepare iron-rebound lactoferrin. The iron contents and protein concentrations of the apo-form lactoferrin and iron-rebound lactoferrin were measured to determine the amounts of iron bound thereto. The measurement was carried out specifically as follows: The apo-form lactoferrin was prepared by dialyzing bLf, 5K-PEG-Lf, and 30k-PEG-Lf against 0.1 M citric acid buffer, pH 2.1, for 24 hours and then dialyzing them against distilled water for another 24 hours. The iron-rebound lactoferrin was prepared by dialyzing the apo-form lactoferrin against a phosphate buffer, pH 7.5, containing 0.001% ammonium iron citrate, 50 mM sodium carbonate and 50 mM sodium chloride for 24 hours, and then dialyzing it against distilled water and then against a phosphate buffer, pH 7.5, containing 50 mM sodium chloride for 24 hours, in order to remove excessive iron ions.

For measuring protein-bound iron ions by the colorimetric method, a serum iron assay kit "Fe C-Test Wako" (Wako Pure Chemical Industries, Ltd.) was used. The ability to bind iron was calculated as the amount of iron bound per 1 mg of protein quantified by the Bradford method, and was expressed as relative activity (%) to the iron binding ability of unmodified bLF as being 100%.

As a result, the iron binding ability of 5K-PEG-Lf which had been modified with PEG was 78.5±4.9% (mean±SE, n=5), while it was 65.6±2.7% (mean±SE, n=6) for 30k-PEG-Lf.

### 7. Evaluation of resistance to pepsin digestion

The purified 30k-PEG-Lf, obtained from the experiment of section 4 above, was digested with pepsin, and the result was compared to the digestion of unmodified bLF.

For the pepsin digestion, to each of unmodified bLF and 30k-PEG-Lf (purified, corresponding to 50µg) pepsin (derived from pig stomach, code No. 165-18713, manufactured by Wako Pure Chemicals Industries, Ltd.) was added to the final concentration of 20 ng/ml, and then reacted in 10 mM HCl at 37°C. 5, 10, 15, 20, 40, 60 and 100 minutes after starting the reaction, an amount corresponding to 5 µg of each protein was sampled by using a pipette and mixed with the same volume of ice-cold 2x sample buffer (100 mM Tris-HCl (pH 6.8), 4% SDS, 20% glycerol, dye (BPB)) to terminate the enzyme reaction.

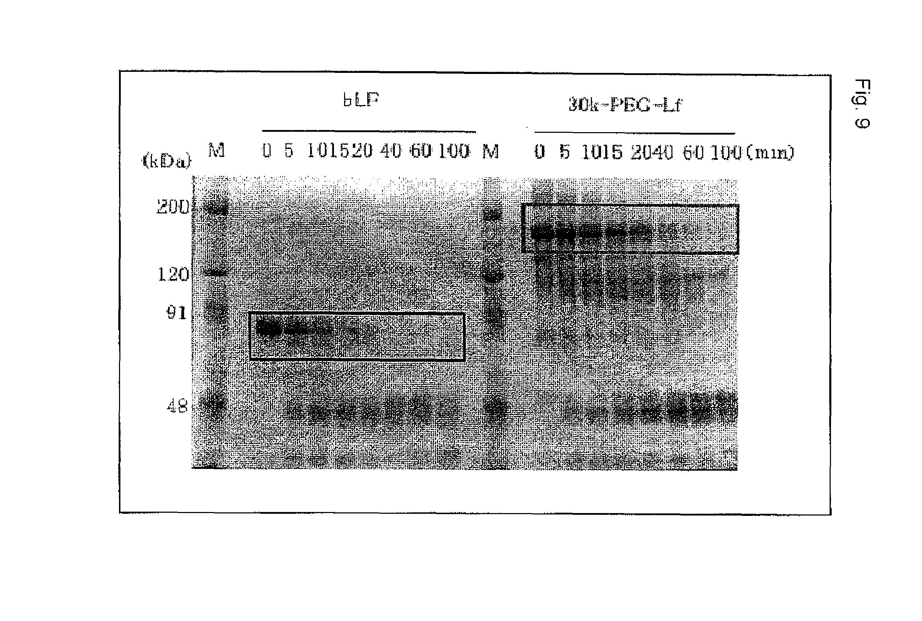

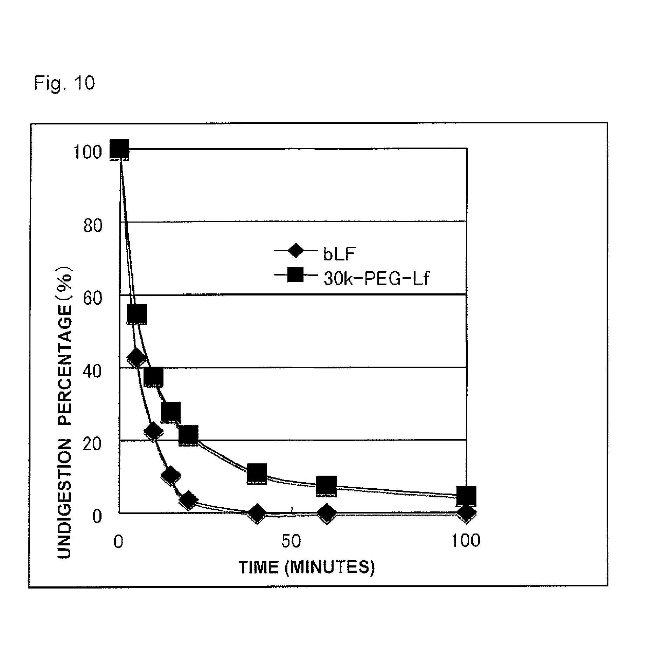

Results are shown in Fig. 9 and Fig.10. Fig. 9 is a photograph of a gel stained with CBB after electrophoresis of each sample on 10% (unreduced) SDS-PAGE. Upon the digestion with pepsin, the unmodified bLF was quickly degraded to smaller molecules, whereas the digestion of 30k-PEG-Lf was limited. From these results, it is demonstrated that the PEGylated LF is less prone to the action by pepsin compared to unmodified bLF.

Fig. 10 shows the results of analysis wherein the electrophoresed image in Fig. 9 was supplied to a scanner and then the density of the bands was analyzed with the software "NIH image" in order to semi-quantitatively show the decomposition with time of intact 30k-PEG-Lf. The density of the band at each time point is shown on the ordinate as relative values to the density at time 0 (minute) as 100%. The time for treatment with each enzyme is shown on the abscissa. The decomposition of the 30k-PEG-Lf by pepsin appeared to be gentle as compared with decomposition of unmodified bLF. From the results above, it is demonstrated that 30k-PEG-Lf is less prone to the action by pepsin compared to unmodified bLF.

This application is based on Japanese Patent Application No. 2008-066626 filed on March 14, 2008, and the entire disclosure and claims of Japanese Patent Application No. 2008-066626 are included in this specification.

## Claims

1. A method of producing a polyethylene glycolated lactoferrin complex, wherein a linear polyethylene glycol (PEG) or a modified product thereof is covalently bonded to lactoferrin via an amide bond, the method comprising:
causing a reaction to occur in a reaction liquid, comprising the lactoferrin and a linear PEG derivative having a para-nitrophenyl group, under conditions that allow formation of an amide group between the para-nitrophenyl group and the lactoferrin.

2. The method according to Claim 1, wherein the reaction step is carried out under the condition of pH 4 to 5.5.

3. The method according to Claim 1 or 2, wherein the reaction step is carried out under the conditions that a molar ratio of the lactoferrin to the linear PEG derivative is 1:1 to 1:5, a final concentration of the lactoferrin is 5 mg/ml to 10 mg/ml, and a reaction time is 24 to 48 hours.

4. A PEGylated lactoferrin complex produced by the method according to any of Claims 1 to 3, wherein a linear polyethylene glycol (PEG) or a modified product thereof is covalently bonded to the lactoferrin via an amide bond.

5. A pharmaceutical composition comprising:
the PEGlyated lactoferrin complex according to Claim 4; and
a therapeutically inert base and/or an additive.

6. The pharmaceutical composition according to Claim 5, which is to be orally administered.
